# FASCICULE DE BREVET EUROPEEN

(11) **EP 0 683 815 B1**
(45) Date de publication et mention de la délivrance du brevet: **25.09.1996**
(21) Numéro de dépôt: 94906245.9
(22) Date de dépôt: 04.02.1994
(51) Int. Cl.: C12M 1/16, C12M 1/06, B01F 7/30

(54) **REACTEUR POUR CONDUIRE DE FACON STERILE DES PROCEDES DE FERMENTATION D'UN PRODUIT A L'ETAT SOLIDE**
REAKTOR ZUR STERILEN DURCHFÜHRUNG VON GÄRUNGSVERFAHREN FÜR EINEN FESTSTOFF
REACTOR FOR STERILE SOLID-STATE FERMENTATION METHODS

(30) Priorité: 10.02.1993 FR 9301455
(43) Date de publication de la demande: 29.11.1995
(73) Titulaire: INSTITUT NATIONAL DE LA RECHERCHE AGRONOMIQUE (I.N.R.A.), 75341 Paris Cédéx 07 (FR)
(72) Inventeur: DURAND, Alain, F-21240 Talant (FR); RENAUD, Raoul, F-21910 Saulon-la-Chapelle (FR); MARATRAY, Jacques, F-21110 Genlis (FR); ALMANZA, Sauveur, F-21160 Marsannay-la-Côte (FR); PELLETIER, André, F-21800 Chevigny-Saint-Sauveur (FR)
(74) Mandataire: Doireau, Marc
(86) Numéro de dépôt international: FR9400134
(87) Numéro de publication internationale: WO9418306

(56) Documents cités:
- EP-A- 0 258 611
- DE-A- 2 559 177
- GB-A- 2 146 979
- BIOTECHNOLOGY AND BIOENGINEERING. vol. 31, no. 5 , Avril 1988 , NEW YORK US pages 476 - 486 A. DURAND ET AL. 'A new pilot reactor for solid-state fermentation: application to the protein enrichment of sugar beet pulp'

## Description

La présente invention concerne un réacteur pour conduire de façon stérile des procédés de fermentation d'un produit à l'état solide, du type comprenant une cuve cylindrique avec une paroi latérale formée d'une double enveloppe, une porte destinée à fermer, de manière étanche, la face d'extrémité inférieure de la cuve, une grille située vers le fond de la cuve, un agitateur rotatif situé au-dessus de la grille, un circuit de circulation d'un gaz au travers de la cuve, et des systèmes de contrôle et de régulation de la température et du taux d'humidité relative du produit en cours de culture.

D'une manière générale, la fermentation d'un produit à l'état solide peut être définie comme un procédé de culture de microorganismes sur et à l'intérieur de particules solides qui peuvent être d'origine végétale (par exemple des pulpes de betteraves, bagasses, paille, son de blé, ...), minérale (perlite, vermiculite, montmorillonite, ...), synthétique (éponges, mousse de polymère, ...), et qui peuvent être imprégnées d'un milieu nutritif liquide plus ou moins complexe.

Des procédés de fermentation à l'état solide sont utilisés depuis fort longtemps pour différentes applications industrielles avec des microorganismes différents (bactéries, levures, champignons par exemple). Parmi ces applications, on peut citer l'alimentation humaine et animale, l'enrichissement protéique de sous-produits agro-alimentaires, la production de divers métabolites (solvants, acides organiques, antibiotiques, hormones végétales, ...) et la production de microorganismes, notamment de champignons filamenteux sous forme mycélienne ou sporulée.

Un réacteur du type précité pour conduire des procédés de fermentation d'un produit à l'état solide est notamment décrit dans le document EP-0 145 536. Toutefois, il est à noter que la face d'extrémité supérieure de la cuve de ce réacteur reste ouverte, que l'aération de la cuve est une aération forcée avec de l'air humide constamment saturé en eau et que le système de régulation du taux d'humidité du produit au cours de la fermentation s'effectue par pulvérisation d'eau à l'aide de buses solidaires d'un pont tournant situé au-dessus de la cuve. Avec un tel système de régulation du taux d'humidité, il faut nécessairement agiter le produit en fermentation au cours de l'aspersion d'eau, ce qui peut gêner le développement de certains microorganismes sensibles aux forces de cisaillement. Enfin, la pulvérisation d'eau à l'intérieur de la cuve est également utilisée pour assurer simultanément la régulation en température du produit en cours de culture.

Un tel réacteur présente par conséquent des limitations tant dans sa structure, que dans le système peu élaboré de régulation en température et en taux d'humidité, ce dernier étant pourtant essentiel pour mener à bien un processus de fermentation dans de bonnes conditions. Il en résulte qu'un tel réacteur présente des performances limitées qui en limitent l'utilisation.

Le but général de l'invention est de concevoir un réacteur pour conduire des procédés de fermentation à l'état solide, qui ne présente pas les limitations du réacteur précité, en permettant notamment de conduire des procédés de fermentation dans des conditions parfaitement stériles, et avec des systèmes de contrôle et de régulation des paramètres relatifs à la température et au taux d'humidité relative du produit qui font intervenir des dispositifs spécifiques propres à chacun de ces paramètres pour les contrôler de manière très précise au cours du processus de fermentation, ces systèmes pouvant être pilotés d'une manière automatique ou manuelle.

A cet effet, l'invention propose un réacteur du type précité et qui est caractérisé en ce que la face d'extrémité supérieure de la cuve est fermée, de manière étanche, par un couvercle, en ce que le circuit de circulation du gaz dans la cuve comprend un circuit d'entrée raccordé sur une ouverture prévue dans la porte de la cuve et un circuit de sortie raccordé sur une ouverture prévue dans le couvercle, le circuit d'entrée comprenant au moins une batterie froide et une batterie chaude pour refroidir et réchauffer le gaz provenant d'une source de distribution, et une enceinte d'humidification pour charger le gaz de plus ou moins d'humidité, en ce que les systèmes de contrôle et de régulation de la température et de l'humidité relative du produit en cours de culture comprennent au moins plusieurs capteurs de température situés au niveau de la cuve, un capteur d'humidité relative situé dans le circuit d'entrée du circuit de circulation du gaz et des moyens de calcul pour traiter les informations fournies par lesdits capteurs pour commander la batterie chaude, la batterie froide, l'enceinte d'humidification et/ou des cycles de rotation de l'agitateur, et en ce qu'il comprend également des dispositifs de stérilisation de la cuve et du circuit de circulation du gaz, de manière à ce que le processus de fermentation puisse être conduit dans des conditions parfaitement stériles.

Selon une autre caractéristique de la cuve du réacteur, la porte est montée pivotante autour d'une charnière fixée à la cuve, et elle supporte la grille au moyen d'entretoises fixées sur la face interne de la porte, un joint torique assurant l'étanchéité en position fermée de la porte.

Selon une autre caractéristique du réacteur conforme à l'invention, l'agitateur est animé d'un mouvement de rotation autour d'un premier axe vertical passant par le centre de la cuve et, simultanément, d'un mouvement de rotation sur lui-même autour d'un second axe vertical passant par le centre de l'agitateur situé sensiblement à mi-distance entre le premier axe vertical et la paroi interne de la cuve.

Selon un mode de réalisation, l'agitateur est constitué de deux barres verticales, entretoisées, espacées l'une de l'autre d'une distance légèrement inférieure au rayon de la cuve et réunies à leurs extrémités par deux tiges de liaison respectivement supérieure et inférieure. Les mouvements de rotation de l'agitateur sont assurés par un groupe moteur dont l'arbre de sortie vertical matérialise le premier axe de rotation, ledit premier arbre étant accouplé en rotation à un système d'entraînement planétaire qui supporte en rotation un second arbre vertical qui matérialise le second axe de rotation et qui est solidaire en rotation de l'agitateur, la transmission de mouvement entre les premier et second arbres étant assurée par un pignon menant, coaxial et solidaire du premier axe, qui engrène un pignon mené, coaxial et solidaire du second arbre.

Selon une autre caractéristique du réacteur conforme à l'invention, l'un des capteurs de température du système de contrôle et de régulation de la température du produit en cours de culture, est constitué par une sonde mobile, à l'aide d'un vérin par exemple, pour mesurer la température au coeur du produit, deux autres capteurs étant respectivement situés en partie haute et en partie basse de la cuve pour mesurer la température du produit à la périphérie de celui-ci, et un dernier capteur pouvant être monté au niveau de la porte de la cuve pour mesurer la température de l'air injecté dans la cuve.

Selon une autre caractéristique du réacteur conforme à l'invention, le contrôle de la température du produit en cours de culture s'effectue à partir des valeurs mesurées par les capteurs de température précités et d'au moins une température de consigne, et sa régulation s'effectue en déclenchant le fonctionnement de la batterie chaude ou de la batterie froide pour réchauffer ou refroidir le gaz à l'entrée de la cuve, en modifiant le débit de gaz par l'intermédiaire du débitmètre massique et/ou en commandant des cycles d'agitation, la batterie froide étant également utilisée pour sécher le produit en fin de fermentation en asséchant le gaz réchauffé par la batterie chaude.

Selon encore une autre caractéristique de l'invention, la double enveloppe de la cuve communique avec l'extérieur par au moins deux ouvertures sur lesquelles se raccordent les entrée-sortie d'un circuit de circulation d'eau, par exemple, comprenant au moins un échangeur de chaleur, une pompe de circulation et une chambre de mesure dans laquelle est logé un capteur de température, et un circuit pour réguler la température de l'eau à une valeur voisine de celle souhaitée pour la fermentation du produit.

Selon une autre caractéristique de l'invention, le contrôle du taux d'humidité relative du produit en cours de culture peut s'effectuer à partir de la valeur mesurée par le capteur d'humidité relative du gaz à l'entrée de la cuve, et sa régulation s'effectue en agissant au niveau du circuit d'humidification pour charger le gaz de plus ou moins d'humidité en fonction de la température de l'eau contenue dans ce circuit qui peut être augmentée au moyen d'une résistance électrique ou diminuée par une circulation d'eau froide dans un serpentin immergé dans l'eau du circuit d'humidification.

D'une manière générale, le contrôle du taux d'humidité relative du produit peut être avantageusement effectué par l'établissement d'un bilan massique, établi par les circuits de calcul, à partir du calcul de la quantité d'eau véhiculée par le gaz grâce aux valeurs données par les capteurs de température et d'humidité relative, ainsi que par le débitmètre massique, par une analyse en continu du gaz carbonique en sortie du réacteur et par le suivi de la masse du produit en cours de culture.

Selon une autre caractéristique du réacteur conforme à l'invention, les dispositifs de stérilisation de la cuve et de l'ensemble des conduits du circuit de circulation du gaz, comprennent au moins un filtre d'entrée monté à la sortie de la source de distribution du gaz située dans le circuit d'entrée du circuit de circulation du gaz, un filtre de sortie monté dans le circuit de sortie du circuit de circulation du gaz, et une entrée de vapeur d'eau située dans le circuit d'entrée du circuit de circulation de gaz.

Selon une autre caractéristique du réacteur conforme à l'invention, le couvercle de la cuve comprend également une ouverture d'entrée de produits à l'intérieur de la cuve, ladite ouverture étant obturable par un bouchon de fermeture traversé par un conduit d'entrée par lequel peuvent être introduites des solutions, ledit conduit étant associé à un dispositif de stérilisation comprenant un conduit relié à une entrée pour injecter de la vapeur d'eau dans le conduit d'entrée et un conduit d'évacuation de ladite vapeur, une vanne étant montée dans le conduit d'entrée pour isoler la cuve dudit dispositif de stérilisation utilisé après chaque introduction d'une solution à l'intérieur de la cuve.

Enfin, selon une autre caractéristique de l'invention, une sonde peut être introduite d'une manière stérile à l'intérieur de la cuve pour prélever un échantillon du produit en cours de culture.

D'une manière générale, les différents domaines d'application de la fermentation à l'état solide cités précédemment dans le cas de procédés non stériles peuvent être traités par le réacteur conforme à l'invention.

Cependant, le fait de maintenir la stérilité au cours du processus de fermentation présente des avantages dont certains sont énumérés ci-dessous :
- application de la fermentation à l'état solide au domaine de l'alimentation humaine et de la pharmacie,
- utilisation de microorganismes nécessitant des durées de culture importantes, de microorganismes génétiquement modifiés et de microorganismes toxiques.

D'autres avantages, caractéristiques et détails de l'invention ressortiront de la description explicative qui va suivre faite en référence aux dessins annexés, donnés uniquement à titre d'exemple, et dans lesquels :
- la figure 1 est une vue en coupe verticale partielle d'un réacteur selon la présente invention,
- la figure 2 est une vue en coupe simplifiée d'une sonde de prélèvement d'un produit en cours de culture à l'intérieur du réacteur,
- la figure 3 est une vue en coupe simplifiée d'une sonde de température déplaçable à l'intérieur du réacteur, et
- la figure 4 illustre schématiquement l'ensemble des systèmes de contrôle et de régulation de la température et du taux d'humidité relative du produit en cours de culture, qui complètent l'équipement du réacteur pour conduire des procédés de fermentation à l'état solide dans des conditions stériles.

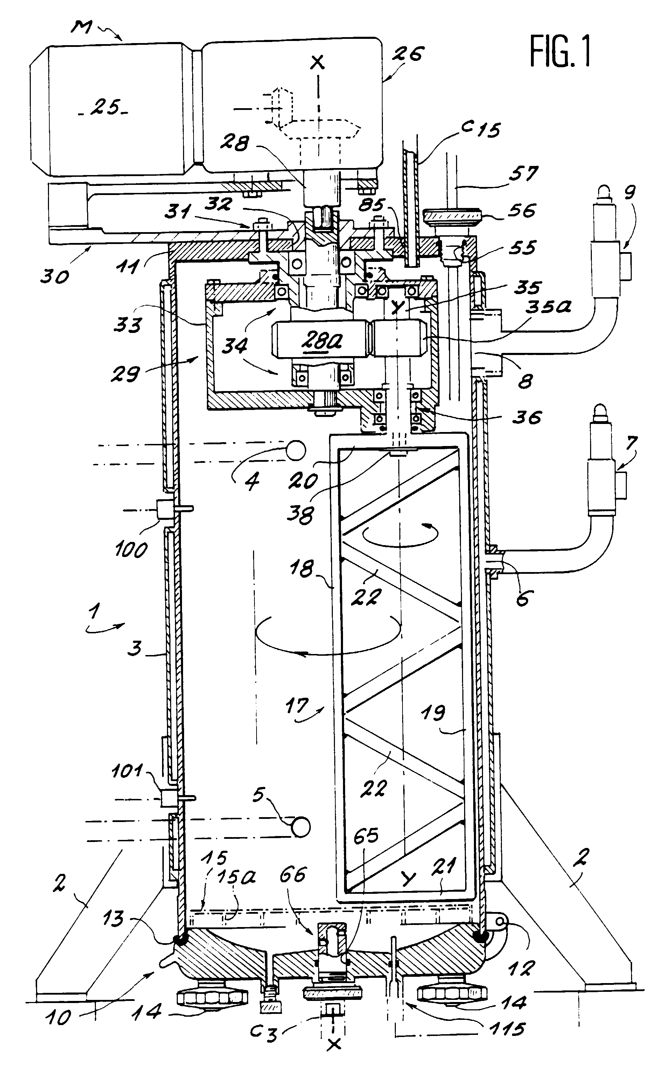

Le réacteur selon l'invention et tel qu'illustré à la figure 1, comprend une cuve 1 de forme cylindrique supportée par au moins deux pieds 2 qui reposent sur le sol ou qui sont solidaires d'un cadre (non représenté) qui repose sur le sol.

La paroi latérale de la cuve 1 est constituée d'une double enveloppe 3 qui s'étend sensiblement sur toute la hauteur de la cuve 1. La paroi externe de la double enveloppe 3 communique avec l'extérieur par deux orifices 4 et 5 sur lesquels viennent se raccorder les entrée/sortie d'un circuit de circulaticn d'eau à l'intérieur de la double enveloppe 3 et qui sera décrit plus loin, et par un troisième orifice 6 pour raccorder une soupape de sécurité 7 qui contrôle la pression à l'intérieur de la double enveloppe 3. Une ouverture latérale 8 traverse la paroi de la cuve 1 pour raccorder une soupape de sécurité 9 qui contrôle la pression à l'intérieur de la cuve 1.

La cuve 1 est fermée de manière étanche, à sa partie inférieure, par une porte 10 et, à sa partie supérieure, par un couvercle 11. La porte 10 est montée pivotante autour d'une charnière 12 fixée à la paroi de la cuve 1. Un joint torique 13 assure l'étanchéité lorsque la porte 10 est fermée et verrouillée au moyen de d'écrous de serrage 14 par exemple. Le couvercle 11 est par exemple constitué par une plaque soudée à la partie supérieure de la cuve 1.

A la partie inférieure de la cuve 1, il est prévu une grille 15 qui est supportée par des entretoises 15a fixées sur la face interne de la porte 10, cette grille 15 prenant une position horizontale lorsque la porte 10 est fermée. Dans la partie centrale de la cuve 1, il est prévu un agitateur rotatif 17 qui est animé d'un mouvement de rotation autour d'un premier axe vertical X-X passant par le centre de la cuve 1, et simultanément, d'un mouvement de rotation sur lui-même autour d'un second axe vertical Y-Y passant par le centre de l'agitateur 17 qui est situé à mi-distance entre l'axe vertical X-X et la paroi interne de la cuve 1. Enfin, à la partie supérieure de la cuve 1, il est prévu un groupe moteur M destiné à entraîner en rotation l'agitateur 17 autour de ses deux axes X-X et Y-Y.

L'agitateur 17 comprend deux barres verticales 18 et 19 espacées l'une de l'autre d'une distance légèrement inférieure au rayon intérieur de la cuve 1. Les deux barres 18 et 19 sont réunies l'une à l'autre par deux tiges d'extrémité horizontales respectivement supérieure 20 et inférieure 21, et par des entretoises intermédiaires inclinées 22 réparties sur la hauteur de l'agitateur 17. D'une manière avantageuse, l'ensemble des barres et des tiges de l'agitateur 17 forme des moyens racleurs.

Le groupe moteur M comprend un moteur 25, du type électrique par exemple, accouplé à un dispositif 26 réducteur de vitesse et de renvoi d'angle dont l'arbre de sortie 28 s'étend verticalement et est accouplé en rotation à un système d'entraînement planétaire 29 relié à l'agitateur 17. Plus précisément, le moteur 25 et le dispositif 26 sont situés à l'extérieur de la cuve 1, et ils sont supportés par une platine 30 fixée au couvercle 11 par des moyens de fixation 31. L'arbre de sortie 28, qui matérialise le premier axe vertical de rotation X-X de l'agitateur 17, est aligné sur un axe passant par le centre de la cuve 1. L'arbre 28 pénètre à l'intérieur de la cuve 1 par une ouverture centrale 32 du couvercle 11. Le système d'entraînement planétaire 29 est constitué d'un boîtier creux 33 logé dans la partie supérieure de la cuve 1, centré sur l'axe vertical X-X et solidaire en rotation de l'arbre 28 par des moyens 34, connus en soi. Le boîtier 33 supporte en rotation un arbre vertical 35 par des moyens 36, connus en soi. Cet arbre 35 matérialise le second axe de rotation Y-Y de l'agitateur 17, et il fait saillie à l'extérieur du boîtier 33 pour être rendu solidaire de l'agitateur 17 par des moyens 38, connus en soi, qui sont montés dans la partie médiane de la tige d'extrémité supérieure 20 de l'agitateur 17. La transmission de mouvement entre les deux arbres 28 et 35 est assurée par un pignon menant 28a, coaxial et solidaire de l'arbre 28, qui engrène un pignon mené 35a, coaxial et solidaire de l'arbre 35.

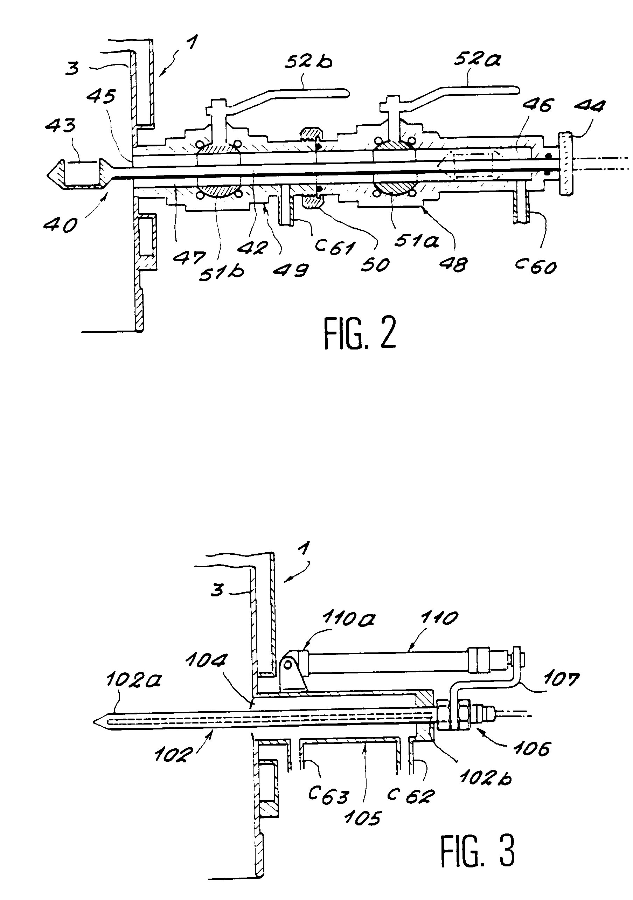

L'équipement de la cuve 1 est complété par une sonde 40 qui permet de prélever, de manière stérile, une fraction du produit en cours de culture. En se reportant à la figure 2, cette sonde 40 est formée d'une tige 42 terminée, à une extrémité, par une coupelle de réception 43 et, à l'autre extrémité, par une collerette 44. Cette sonde 40 peut être introduite à l'intérieur de la cuve 1 par une ouverture latérale 45 située à mi-hauteur de la cuve 1, et après avoir traversé une chambre 46 prolongée par un canal 47 qui débouche dans l'ouverture 45. La chambre 46 est formée dans le corps d'une première pièce 48, et le canal 47 est formé dans le corps d'une seconde pièce 49 fixée sur la cuve 1 autour de l'ouverture 45. La première pièce 48 est fixée de manière amovible à la seconde pièce 49 au moyen d'un écrou 50. Des premiers moyens d'obturation 51a manoeuvrés par un levier 52a sont logés à l'intérieur de la chambre 46 vers l'extrémité de celle-ci qui débouche dans le canal 47, et des seconds moyens d'obturation 51b manoeuvrés par un levier 52b sont logés à l'intérieur du canal 47. A titre d'exemple, chacune des pièces 48 et 49 peut être constituée par une vanne quart de tour à boisseau sphérique, ce dernier constituant chaque moyen d'obturation précité 51a et 51b.

Le couvercle 11 de la cuve 1 (figure 1) est percé d'une ouverture 55 dans laquelle se visse un bouchon de fermeture 56 traversé par un conduit d'entrée 57 pour l'introduction de solutions dans la cuve 1.

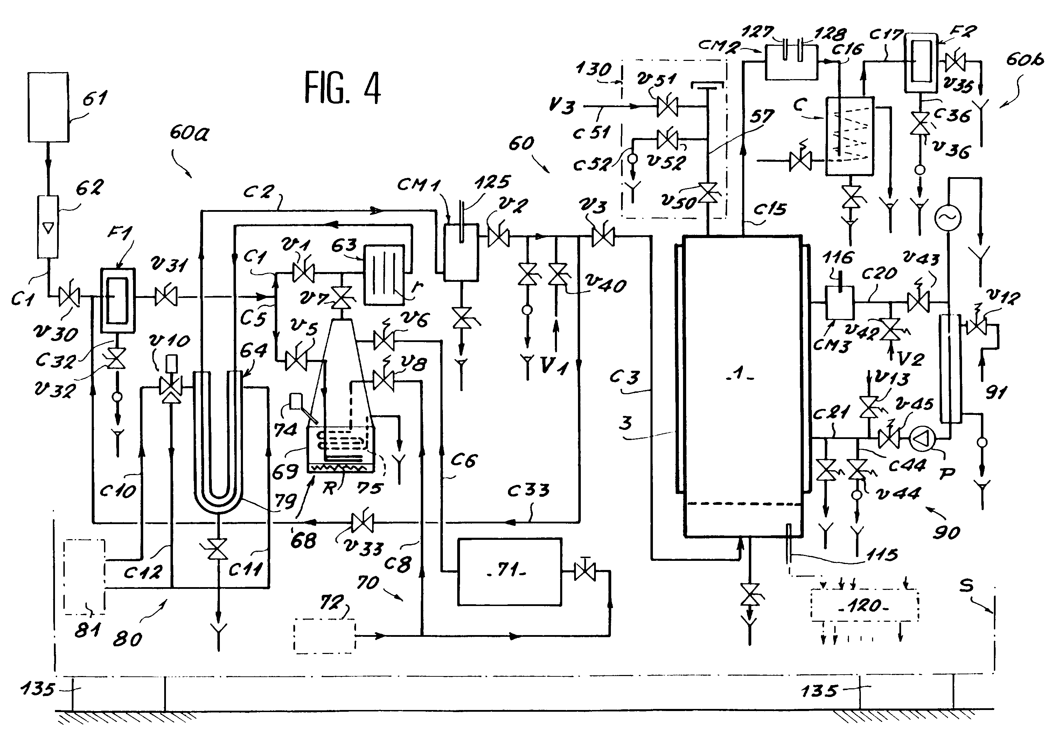

Le réacteur est équipé d'un circuit de circulation 60 d'un gaz, tel que l'air, au travers de la cuve 1. Ce circuit 60, illustré schématiquement à la figure 4, se scinde en un circuit d'entrée 60a situé en amont de la cuve 1 et en un circuit de sortie 60b situé en aval de la cuve 1, en considérant le sens d'écoulement de l'air.

Le circuit d'entrée 60a comprend un conduit c1 dont une extrémité est raccordée sur la sortie d'une source de distribution d'air 61 avec interposition d'un débitmètre massique 62 pour réguler et mesurer le débit d'air injecté dans la cuve 1. L'autre extrémité du conduit cl est raccordée sur l'entrée d'une batterie chaude 63 avec interposition d'une électro-vanne v1. La batterie chaude 63 comporte plusieurs résistances r reliées à une source de tension (non représentée) et destinées à réchauffer l'air. Un conduit c2 raccordé, à une extrémité, sur la sortie de la batterie chaude 63, traverse une batterie froide 64 et est raccordé, à son autre extrémité, sur l'entrée d'une chambre de mesure CM1. Un conduit c3 raccordé, à une extrémité, sur la sortie de la chambre CM1 est raccordé à son autre extrémité, sur une entrée de la cuve 1 avec interposition de deux électro-vannes v2 et v3. Cette entrée est formée par une ouverture centrale 65 (figure 1) ménagée dans la porte 10. Un diffuseur d'air 66 est branché sur cette ouverture 65, côté intérieur de la porte 10, pour assurer une diffusion de l'air au travers de toute la surface de la grille 15.

Un circuit d'humidification 68 de l'air est placé en amont de la batterie chaude 63 et il comprend une enceinte fermée 69. Un conduit c5 débouche, à une extrémité, dans le fond de l'enceinte 69, et il est raccordé, à son autre extrémité, au conduit cl précité en un point situé en amont de l'électro-vanne v1 et avec interposition d'une électro-vanne v5 à membrane biologique. L'enceinte 69 est alimentée en eau stérile par un circuit 70 comprenant un ensemble 71 constitué d'un pré-filtre, d'un adoucisseur et d'un filtre stérilisant, par exemple. Un conduit c6 est branché, à une extrémité, sur la sortie de l'ensemble 71, alors que son autre extrémité débouche à l'intérieur de l'enceinte 69 avec interposition d'une électro-vanne v6 commandée par un capteur 74 pour maintenir un niveau d'eau sensiblement constant à l'intérieur de l'enceinte 69. Un conduit c7 est raccordé, à une extrémité, à l'entrée de l'ensemble 71 et, à son autre extrémité, à une source 72 d'eau non-stérile. Enfin, un serpentin 75 est immergé dans l'eau stérile contenue dans l'enceinte 69, et dans lequel circule de l'eau non-stérile prélevée de la source 72 par un conduit c8 avec interposition d'une électro-vanne v8. L'eau qui circule dans le serpentin 75 permet d'abaisser la température de l'eau stérile contenue dans l'enceinte 69, alors qu'une résistance R située dans le fond de cette enceinte 69 permet de relever ladite température.

La batterie froide 64 est constituée par une enceinte fermée 79, en forme de U, associée à un circuit 80 de circulation d'une eau glycolée, par exemple, provenant d'un groupe froid 81 qui alimente l'enceinte 79 par une vanne motorisée v10 à 3 voies. Un conduit c10 achemine l'eau à une entrée de l'enceinte 79 au travers de deux voies de la vanne v10. Un conduit c11 est branché sur une sortie de l'enceinte 79 pour ramener l'eau vers le groupe froid 81, et un conduit c12 est branché entre la troisième voie de la vanne v10 et le conduit c11 pour court-circuiter l'enceinte 79.

Le circuit de sortie 60b du circuit 60 de circulation d'air au travers de la cuve 1, comprend un conduit c15 qui est raccordé, à une extrémité, autour d'une ouverture 85 du couvercle 11 de la cuve 1 (figure 1) et, à l'autre extrémité, à l'entrée d'une chambre de mesure CM2 dont la sortie est reliée par un conduit c16 à l'entrée d'un condenseur C dont la sortie est reliée à l'extérieur par un conduit c17.

En se reportant toujours à la figure 4, un circuit 90 assure une circulation d'eau à l'intérieur de la double enveloppe 3 de la cuve 1. Ce circuit 90 est un circuit fermé comprenant un échangeur de chaleur 91 relié par un conduit c20 à l'entrée d'une chambre de mesure CM3 dont la sortie est raccordée autour de l'orifice d'entrée 4 de la double enveloppe 3, et par un conduit c21 à l'orifice de sortie 5 de la double enveloppe 3 avec interposition d'une pompe de circulation P.

Le réacteur est équipé d'un système de contrôle et de régulation de la température du produit en cours de culture.

Ce contrôle est assuré par deux capteurs de température supérieur 100 et inférieur 101 (figure 1) fixés sur la paroi latérale de la cuve 1 pour mesurer en permanence la température à la périphérie du produit, et par un capteur de température intermédiaire 102 déplaçable à l'intérieur de la cuve pour mesurer en permanence la température au coeur du produit en culture.

En se reportant plus particulièrement à la figure 3, le capteur intermédiaire 102 est constitué par une sonde dont une extrémité 102a pénètre à l'intérieur de la cuve 1 par une ouverture latérale 104 après avoir traversé un élément tubulaire 105 rapporté autour de l'ouverture 104. L'autre extrémité 102b de la sonde qui fait saillie à l'extérieur de l'élément 105 est fixée, par des moyens 106, à l'extrémité d'une tige de liaison 107 dont l'autre extrémité est fixée à l'extrémité d'une tige de piston d'un vérin 110, du type hydraulique par exemple. Le corps du vérin 110 s'étend parallèlement à l'élément tubulaire 105 et est fixé sur celui-ci au moyen d'une chape 110a, par exemple. L'actionnement du vérin 110 permet de déplacer le capteur 102 de manière à ce que son extrémité 102a puisse au moins atteindre le centre de 0 la cuve 1.

Le contrôle de la température du produit en cours de culture peut également s'effectuer à partir d'un capteur 115 fixé sur la porte 10 de la cuve 1 (figure 1) pour mesurer en permanence la température de l'air à l'entrée de la cuve 1.

Les capteurs de température 100, 101, 102 et 115 sont reliés à un calculateur central 120 (figure 4) piloté par un logiciel enregistré dans une mémoire pour traiter toutes les valeurs mesurées par ces capteurs en fonction de valeurs de consignes prédéterminées, de manière à assurer la régulation de la température du produit en cours de culture sur la base des températures du produit prélevées par les capteurs 100, 101 et 102 ou de la température de l'air à l'entrée de la cuve. Cette régulation est effectuée dans les deux cas en jouant sur la température de l'air à l'entrée de la cuve 1 par :
- la mise en circuit d'une ou plusieurs des résistances r de la batterie chaude 63 pour réchauffer l'air avant son entrée dans la cuve 1,
- la commande de l'électro-vanne v10 de la batterie froide 64 pour faire circuler l'eau provenant du groupe froid 81 à l'intérieur de l'enceinte 79 de la batterie pour refroidir l'air avant son entrée dans la cuve 1,
- la commande du débitmètre massique 62 pour augmenter ou diminuer le débit d'air dans le circuit de circulation d'air 60 au travers de la cuve 1, et/ou
- l'actionnement du groupe moteur M pour commander des cycles d'agitation de l'agitateur rotatif 17 à l'intérieur de la cuve 1.

D'une manière générale, ces actions pour assurer la régulation de la température du produit en culture peuvent être hiérarchisées au niveau du logiciel enregistré dans le calculateur 120 en fonction par exemple du milieu et du microorganisme qui sont utilisés. Ainsi, le manipulateur peut par exemple donner la priorité d'une action sur une augmentation ou une diminution du débit d'air et, si cela n'est pas suffisant, commander une action sur la batterie froide ou chaude, puis en dernier ressort en commandant des cycles d'agitation.

En parallèle, la température de l'eau qui circule dans la double enveloppe 3 est contrôlée par un capteur de température 116 situé dans la chambre de mesure CM3 du circuit 90 de circulation d'eau et relié au calculateur 120. La régulation de cette température est effectuée par injection de vapeur à l'intérieur de l'échangeur de chaleur 91 par l'intermédiaire d'une électro-vanne v12 lorsque l'on veut réchauffer l'eau et par injection d'eau froide dans le conduit c21 au travers d'une électro-vanne v13 lorsque l'on veut refroidir l'eau. La température de l'eau est généralement voisine de celle à laquelle doit se dérouler la culture du produit à l'intérieur de la cuve 1.

Le réacteur est également équipé d'un système de contrôle et de régulation du taux d'humidité relative du produit en culture à l'intérieur de la cuve 1. Ce système comprend notamment un capteur 125 monté dans la chambre CM1 du circuit d'entrée 60a du circuit 60 de circulation d'air et qui est également relié au calculateur 120.

Le capteur d'humidité 125 permet de contrôler la teneur en eau du gaz injecté dans la cuve 1, et la régulation du taux d'humidité relative du produit en cours de culture peut être pilotée par le calculateur 120 à partir des valeurs fournies par le capteur 125 et d'une valeur de consigne, en agissant au niveau du circuit d'humidification 68 pour charger l'air de plus ou moins d'humidité avant son entrée dans la cuve 1.

D'une manière avantageuse, cette régulation peut être effectuée :
- soit par l'établissement d'un bilan massique effectué par le calculateur 120 à partir du calcul de la quantité d'eau véhiculée par l'air circulant dans le circuit de circulation 60 grâce aux valeurs données par les capteurs de température et d'humidité relative, ainsi que par le débitmètre 62, par une analyse en continu du gaz carbonique en sortie du réacteur et par le suivi de la masse du produit en cours de culture,
- soit par la programmation au niveau du calculateur 120 d'un profil prédéterminé d'évolution des consignes de température, d'humidité relative et de débit d'air, profil déterminé lors d'expériences précédentes.

Dans tous les cas, la régulation du taux d'humidité est commandée par le calculateur 120 qui assure :
- soit la mise en circuit de la résistance R placée au fond de l'enceinte 69 du circuit d'humidification 68 pour réchauffer l'eau,
- soit la commande de l'électro-vanne v8 pour assurer une circulation d'eau froide dans le serpentin 75 immergé dans l'enceinte d'humidification 69 pour refroidir l'eau,
de manière à ce que l'air se charge plus ou moins d'humidité suivant la température de l'eau.

Pour des raisons de simplification, les différentes liaisons entre le calculateur, les différents capteurs et les électro-vannes n'ont pas été représentées en détail.

Il est à noter que deux capteurs de température 127 et de pression 128 sont montés dans la chambre CM2 du circuit de sortie 60b du circuit de circulation d'air 60. Le capteur de température 127 peut être utilisé pour contrôler la température du produit en culture dans la cuve 1, et le capteur de pression 128 permet de contrôler la pression à l'intérieur de la cuve 1 et entraîner, si nécessaire, une procédure d'arrêt du réacteur. Ces capteurs 127 et 128 sont également reliés au calculateur central 120.

L'équipement du réacteur est complété par des dispositifs de stérilisation de manière à conduire un processus de fermentation à l'état solide dans des conditions parfaitement stériles, dispositifs qui sont décrits ci-après.

Un filtre F1 est intercalé dans le conduit cl du circuit d'entrée 60a du circuit 60 de circulation d'air. Ce filtre F1 est monté entre le débitmètre 62 et la batterie chaude 63, avec deux électro-vannes v30 et v31 placées en amont et en aval du filtre F1. Un conduit d'évacuation c32, avec interposition d'une électro-vanne v32, fait communiquer le filtre F1 avec l'extérieur.

Un conduit de dérivation c33 dans lequel est intercalé une électro-vanne v33 débouche, à une extrémité, dans le conduit c1 entre la vanne v30 et l'entrée du filtre F1 et, à l'autre extrémité, entre les deux vannes v2 et v3 placées dans le conduit c3 qui débouche dans la porte 10 de la cuve 1. Ce conduit de dérivation c33 permet de court-circuiter la batterie chaude 63, la batterie froide 64 et le circuit d'humidification lors d'une opération de stérilisation.

Un filtre F2 est intercalé dans le conduit c17 à la sortie du condenseur C du circuit de sortie 60b du circuit 60 de circulation d'air, avec interposition d'une électro-vanne v35 en aval du filtre F2. Un conduit d'évacuation c36 avec interposition d'une électro-vanne v36 fait communiquer le filtre F2 avec l'extérieur.

Une entrée V1 de vapeur d'eau, avec interposition d'une électro-vanne v40, débouche dans le conduit c3 du circuit d'entrée 60a du circuit de circulation d'air 60 entre les deux vannes v2 et v3, pour effectuer la stérilisation du filtre F1 par le conduit c33, des conduits c1, c5 et c3 (la vanne v30 restant fermée) du circuit d'entrée 60a, de la cuve 1, des conduits c15, c16 et c17 et du filtre F2 du circuit de sortie 60b.

Une entrée V2 de vapeur d'eau, avec interposition d'une électro-vanne v42, débouche dans le conduit c20 du circuit 90 de circulation d'eau dans la double enveloppe 3 de la cuve 1. Cette entrée de vapeur d'eau s'effectue entre la chambre CM3 et une électro-vanne v43 interposée dans le conduit c20. Un conduit d'évacuation c44 de la vapeur d'eau, après circulation dans la double enveloppe 3, se raccorde au conduit c21 avec interposition d'une électro-vanne v44, alors qu'une électro-vanne v45 est placée dans le conduit c21 entre le point de raccordement du conduit c44 et la pompe de circulation P.

Comme cela a été décrit en référence à la figure 1, la cuve 1 comporte un conduit d'entrée 57 au niveau de son couvercle 11 pour introduire des solutions à l'intérieur de la cuve. Ce conduit 57 est également associé à un circuit de stérilisation d'entrée 130 qui comprend une électro-vanne v50 interposée dans le conduit, et deux circuits de dérivation c51 et c52. Le circuit de dérivation c51 est relié à une entrée V3 de vapeur d'eau avec interposition d'une électro-vanne v51, alors que le conduit de dérivation c52 communique avec l'extérieur avec interposition d'une électro-vanne v52.

Des circuits de stérilisation sont également prévus au niveau de la sonde de prélèvement 40 et de la sonde de température 102.

En se reportant à la figure 2, il est prévu un conduit d'entrée c60 qui débouche dans la chambre 46, et un conduit de sortie c61 qui débouche dans le canal 47, conduits qui sont utilisés pour injecter et évacuer de la vapeur d'eau, dans des conditions qui seront précisées plus loin.

D'une manière similaire, en se reportant à la figure 3, le conduit 105 qui est traversé par la sonde 102 peut être stérilisé par injection de vapeur au travers d'un conduit d'entrée c62 et évacuation par un conduit de sortie c63, ces deux conduits débouchant à l'intérieur de l'élément tubulaire 105, vers les deux extrémités de celui-ci.

Selon un mode préférentiel de réalisation du circuit d'entrée 60a du circuit 60 de circulation de l'air, comme cela est représenté sur la figure 4, il est à noter que la batterie froide 64 se situe après la batterie chaude 63, en considérant le sens de l'écoulement de l'air. Cette disposition permet d'assurer une meilleure régulation de la température et de l'humidité relative de l'air à l'entrée de la cuve 1.

Enfin, selon un mode de réalisation préférentiel, la cuve 1 et l'ensemble des circuits, dispositifs et systèmes dont elle est équipée sont solidaires d'un cadre vertical S matérialisé schématiquement en traits mixtes sur la figure 4. Ce cadre vertical S repose avantageusement sur le sol par l'intermédiaire de plusieurs capteurs de masse 135 qui permettent de mesurer en continu la masse du produit en cours de culture pour établir le bilan massique dont il a été précédemment fait référence pour la régulation du taux d'humidité relative du produit en cours de culture.

A titre d'exemple, le diamètre intérieur de la cuve 1 est de 360 mm et sa paroi latérale s'étend sur une hauteur de l'ordre de 850 mm, ce qui lui donne une capacité d'environ 90 litres. Le moteur 25 de l'agitateur 17 a par exemple une puissance de 1500 W, et l'agitateur 17 peut être entraîné en rotation autour de l'axe X-X à une vitesse de l'ordre de 54 t/min et autour de l'axe Y-Y à une vitesse de l'ordre de 18 t/min.

Il va être maintenant décrit le fonctionnement général du réacteur tel que précédemment décrit en s'appuyant sur un exemple de culture.

D'une manière générale, un certain nombre de séquences doivent être respectées avant le déroulement de la culture proprement dite en milieu solide, pour que cette culture puisse se dérouler dans des conditions parfaitement stériles.

Une première séquence consiste à effectuer une stérilisation dite à vide de la cuve et de l'ensemble de son équipement. Cette opération s'effectue en injectant de la vapeur d'eau par l'entré V1 du circuit d'entré 60a du circuit 60 de circulation d'air La vanne v40 associée à l'entrée V1 est par conséquent ouverte, les vannes v2 et v3 du conduit c3 sont respectivement fermée et ouverte, la vanne v33 du conduit de dérivation c33 est ouverte, et les vannes v1, v5 et v30 étant fermées (la vanne v31 restant ouverte). Dans ces conditions, de la vapeur d'eau est injectée par l'intermédiaire du conduit c3 à l'intérieur de la cuve 1 en pénétrant dans celle-ci par l'ouverture d'entrée 65 prévue dans la porte 10. La vapeur d'eau est ensuite évacuée de la cuve 1 par le conduit c15 du circuit de sortie 60b du circuit 60 de circulation d'air. La vapeur d'eau, en sortie de la cuve 1, traverse la chambre de mesure CM2, le condenseur C et le filtre de sortie F2, avant d'être évacuée vers l'extérieur par l'intermédiaire du conduit c36 et de la vanne v36 qui est en position d'ouverture.

La vapeur d'eau injectée par l'entrée V1 circule également à travers le conduit de dérivation c33 du circuit d'entrée 60a du circuit 60 de circulation d'air pour stériliser le filtre d'entrée F1 avant d'être évacuée vers l'extérieur par le conduit c32 et la vanne v32 qui est en position d'ouverture. La mise en circuit du conduit de dérivation c33 et la fermeture des vannes V31, en sortie du filtre d'entrée F1, et la vanne v2, en sortie de la chambre de mesure CM1, permettent de court-circuiter la batterie chaude 63, la batterie froide 64 et l'humidificateur 68. Cette partie du circuit d'entrée 60a peut être stérilisée à part, par exemple au démarrage de l'installation par une solution de formol. Par la suite, cette partie est stérilisée en permanence, étant donné qu'elle reçoit de l'air stérilisé par le filtre d'entrée F1, et de la vapeur d'eau stérile produite à partir de l'humidificateur 68.

La vapeur d'eau injectée dans la cuve 1 pénètre également à l'intérieur du conduit d'entrée 57 prévu au niveau du couvercle 11 de la cuve 1 pour y introduire des solutions. La vanne v50 placée dans ce conduit 57 est ouverte ainsi que la vanne v52 du conduit d'évacuation c52, alors que la vanne v51 du conduit c51 est fermée. Dans ces conditions, la vapeur d'eau qui circule dans le conduit 57 permet de stériliser celui-ci avant de s'évacuer par le conduit c52.

Enfin, le circuit 90 de circulation d'eau à l'intérieur de la double enveloppe 3 de la paroi latérale de la cuve est stérilisé par injection de vapeur par l'entrée V2 et la vanne v42 dans le conduit c20. La vapeur d'eau se dirige vers la chambre de mesure CM3 par suite de la fermeture de la vanne v43 placée dans ce conduit c20. La vapeur d'eau pénètre à l'intérieur de la double enveloppe 3 par l'entrée 4 et s'évacue par la sortie 5 de la cuve 1 qui communique avec le conduit c21 avant de s'évacuer par la vanne v44 ouverte du conduit d'évacuation c44, les vannes v13 et v45 étant fermées.

Cette opération de stérilisation est effectuée pendant une durée de 30 minutes environ et à une température de l'ordre de 120° par exemple.

La séquence suivante a pour objet de sécher les filtres d'entrée F1 et de sortie F2. Pour cela, la source 61 de distribution d'air est activée pour sécher dans un premier temps le filtre d'entrée F1. L'air passe dans le conduit cl, traverse la vanne ouverte v30 pour pénétrer à l'intérieur du filtre F1 et ressortir par le conduit d'évacuation c32 et la vanne v32 ouverte, alors que la vanne v31, à la sortie du filtre F1, est fermée, ce qui permet d'isoler le filtre F1 du reste du circuit d'entrée 60a du circuit 60 de circulation d'air.

Ensuite, la vanne v32 est fermée et la vanne v31 est ouverte pour que l'air traverse le filtre d'entrée F1 et parvienne jusqu'au filtre de sortie F2 situé dans le circuit de sortie 60b du circuit 60 de circulation d'air. Concrètement, après avoir traversé le filtre F1, l'air est acheminé par le conduit cl jusqu'à la batterie chaude 63 (vanne v1 ouverte et vannes v5 et v7 fermées pour isoler le circuit d'humidification 68). Après avoir traversé la batterie chaude 63, l'air est acheminé par le conduit c2 au travers de la batterie froide 64 jusqu'à la chambre de mesure CM1. L'air est ensuite acheminé par le conduit c3 jusqu'à l'entrée de la cuve 1 (vannes v2 et v3 ouvertes, et vanne v40 fermée). L'air est ensuite évacué de la cuve 1 par le conduit c15 et est acheminé au travers de la chambre de mesure CM2 et le condenseur C, jusqu'à l'entrée du filtre F2, avant d'être évacué par le conduit c36 et la vanne v36 ouverte, la vanne v35 restant fermée.

A titre indicatif, le séchage des filtres d'entrée F1 et de sortie F2 s'effectue pendant une durée de cinq minutes environ.

La suite du fonctionnement du réacteur va être décrite dans le cas de la production de spores de "trichoderma harzianum" sur des pulpes de betterave, par exemple, qui forment le milieu solide.

Concrètement, des pulpes de betterave sèches sont introduites dans la cuve 1 par l'ouverture d'entrée 55 du couvercle 11, le bouchon de fermeture 56 de cette ouverture ayant été enlevé. On ajoute ensuite un complément minéral sous forme d'une solution composée de CACL2 (1 g/l) de KH2PO4 (1p/l), de (NH4)2SO4 (3,25 g/l) et d'urée (0,75g/l). A titre d'exemple, on introduit dans la cuve 1 huit kilos de pulpes de betterave sèches et douze litres de complément minéral.

Une fois le milieu de culture introduit dans la cuve 1, on procède à sa stérilisation. Cette opération s'effectue globalement dans les mêmes conditions que l'opération de stérilisation à vide décrite précédemment. A titre indicatif, cette opération dure pendant environ 30 minutes, s'effectue à une température de l'ordre de 120°, avec agitation permanente du milieu à l'aide de l'agitateur rotatif 17.

Une fois cette opération de stérilisation du milieu effectuée, on procède à nouveau à une opération de séchage des filtres d'entrée F1 et de sortie F2, d'une manière semblable à celle décrite précédemment.

Ensuite, on procède à une opération de refroidissement rapide pour abaisser la température à l'intérieur de la cuve 1. Cette opération de refroidissement est réalisée en actionnant le circuit 90 pour assurer une circulation forcée d'eau froide dans la double enveloppe 3, jusqu'à ce que la température de la cuve 1 soit abaissée à une température de consignes fixée pour la fermentation. Pour cela, de l'eau froide est injectée par la vanne v13 dans le conduit c21, les vannes v43 et v45 étant ouvertes, alors que la vanne v44 est fermée. Pendant cette opération de refroidissement, l'agitateur 17 est en fonctionnement, et on injecte également de l'air à l'intérieur de la cuve 1 afin d'éviter une mise sous vide. Ensuite, on procède à l'inoculation d'un fermenteur qui est préparé à partir d'une solution concentrée de spores de "trichoderma harzianum", de manière à introduire 210⁷ spores/gramme de pulpes de betterave sèches. Cette solution préparée selon des techniques connues est ensuite mélangée à un volume d'eau stérile afin d'obtenir la concentration voulue en fonction de la masse de pulpes de betterave sèches préalablement introduite dans la cuve 1. L'inoculum est ensuite introduit stérilement à l'intérieur de la cuve 1 par l'intermédiaire du conduit 57 qui traverse le bouchon 56 du couvercle 11 de la cuve 1. Une fois l'inoculation effectuée, la vanne v50 située dans le conduit d'entrée 57 est fermée. Ensuite, on prend la précaution de restériliser ce conduit 57 en introduisant de la vapeur d'eau par l'entrée V3 avec évacuation par le conduit c52, les vannes v51 et v52 étant alors ouvertes et la vanne v50 fermée.

Il est à noter que lors de l'introduction de l'inoculum dans la cuve, l'agitateur 17 est en action pour obtenir une bonne répartition du microorganisme dans toute la masse du milieu solide.

La culture peut alors commencer sous le contrôle des systèmes de régulation de la température et du taux d'humidité relative du produit en cours de culture, ces régulations étant primordiales dans le cas d'une fermentation en milieu solide.

D'une manière générale, les capteurs de température 100 et 101 permettent de connaître à tout instant la température à la périphérie de la masse du produit en culture, alors que la sonde de température 102 permet de connaître la température au coeur du produit. Pour cela, le vérin 110 est actionné, ce qui implique que l'agitateur 17 n'est pas en rotation. Le capteur de température 115 situé au niveau de la porte 10 de la cuve 1 permet également de contrôler la température du produit en mesurant la température de l'air injecté à l'intérieur de la cuve 1.

Le logiciel enregistré dans la mémoire du calculateur 120 peut assurer le pilotage de la régulation de la température de la culture en prenant en compte les informations recueillies soit à partir de la sonde 102 qui mesure la température au coeur du produit, soit à partir du capteur 115 qui mesure la température de l'air à l'entrée de la cuve 1. Dans les deux cas, le calculateur 120, après traitement de ces informations, peut agir :
- au niveau de la batterie chaude 63 pour augmenter ou diminuer le nombre de résistances électriques r qui sont mises sous tension pour augmenter ou abaisser la température de l'air injecté dans la cuve 1,
- au niveau de la batterie froide 64 pour refroidir l'air injecter à l'intérieur de la cuve 1 en commandant l'électro-vanne v10,
- au niveau du débitmètre 62 pour augmenter ou diminuer le débit d'air injecté à l'intérieur de la cuve 1, et/ou
- au niveau du groupe moteur M pour commander des cycles d'agitation de l'agitateur 17.

Comme cela a été précédemment indiqué, ces différentes actions peuvent être hiérarchisées en mettant par exemple la priorité sur une augmentation ou une diminution du débit d'air puis, si cela n'est pas suffisant, provoquer une action de la batterie froide ou de la batterie chaude, puis en dernier lieu, commander des cycles d'agitation.

En parallèle, on effectue également une régulation de la teneur en eau du milieu de culture pendant la fermentation, cette régulation pouvant être avantageusement effectuée soit en établissant un bilan massique tout au long de la fermentation, soit à partir d'un profil programmé à partir d'expériences antérieures, comme indiqué précédemment.

D'une manière générale, cette régulation s'effectue en agissant soit sur la résistance chauffante R du circuit d'humidification 68, soit en commandant l'ouverture de la vanne v8 du circuit de circulation d'eau froide 70 dans le serpentin 75 situé dans l'enceinte d'humidification 69.

Au cours de la culture du produit, il est 0 possible d'en prélever une partie par l'intermédiaire de la sonde de prélèvement 40, l'agitateur 17 n'étant pas en action. Pour cela, on ouvre les deux moyens obturateurs 51a et 52a de manière à pouvoir introduire la sonde 40 au travers de la chambre 46 et du canal 47, jusqu'à ce que le collet 44 de la tige 42 de la sonde 40 vienne obturer l'extrémité ouverte de la chambre 46. En pénétrant dans la masse du produit, la sonde en prélève une partie par l'intermédiaire de la coupelle 43, et l'on retire ensuite la sonde 40 jusqu'à amener celle-ci à l'intérieur de la chambre 46. On ferme ensuite les deux obturateurs 51a et 52a en agissant respectivement sur les deux leviers 51b et 52b. On désolidarise ensuite les deux pièces 48 et 49 par l'intermédiaire de l'écrou 50, ce qui permet de conserver le produit extrait à l'intérieur de la chambre 46 et de le maintenir dans une atmosphère stérile avant d'être examiné.

Dans l'exemple considéré ici, la culture est maintenue pendant environ 80 heures.

A la fin de la culture, l'ensemble du milieu de culture peut être séché directement à l'intérieur de la cuve 1 par une circulation d'air sec à la température souhaitée. Dans cet exemple, la culture est séchée à une température de 30° environ avec un air n'excédant pas 10 % d'humidité relative, et avec une agitation intermittente toutes les heures, pendant 5 minutes. Lors de la procédure de séchage, l'air stérile passe directement sur les batteries chaude 63 et froide 64, sans passer par le circuit d'humidification 68.

Toutes les séquences décrites précédemment pour conduire un processus de fermentation à l'état solide sont pilotées par le calculateur 120, mais elles peuvent être commandées en totalité ou en partie seulement de manière manuelle par un opérateur.

D'une manière générale, il est prévu un ensemble de sécurités, comme celles déjà assurées par les soupapes 7 et 9, pour éviter tout déclenchement inopiné d'une séquence pendant le déroulement d'une autre séquence, ces sécurités pouvant être gérées par le logiciel du calculateur 120.

L'invention n'est nullement limitée au mode de réalisation décrit précédemment et donné à titre d'exemple. Il est en effet possible d'équiper la cuve 1 avec un couvercle 11 du type pivotant par exemple, et de prévoir un hublot au niveau de la cuve 1 pour examiner le produit en cours de culture et modifier éventuellement le déroulement du processus de fermentation.

## Revendications

1. Réacteur pour conduire de façon stérile des procédés de fermentation d'un produit à l'état solide, du type comprenant une cuve cylindrique à paroi latérale formée d'une double enveloppe, une porte destinée à fermer, de manière étanche, la face d'extrémité inférieure de la cuve, une grille située vers le fond de la cuve, un agitateur rotatif situé au-dessus de la grille, un circuit de circulation d'un gaz au travers de la cuve, et des systèmes de contrôle et de régulation de la température et du taux d'humidité du produit en cours de culture, caractérisé en ce que la face d'extrémité supérieure de la cuve (1) est fermée, de manière étanche, par un couvercle (11), en ce que le circuit (60) de circulation du gaz au travers de la cuve (1) comprend un circuit d'entrée (60a) raccordé sur une ouverture (65) prévue dans la porte (10) et un circuit de sortie (60b) raccordé sur une ouverture (85) prévue dans le couvercle (11), le circuit d'entrée (60a) comprenant en série au moins une batterie chaude (63) et une batterie froide (64) pour réchauffer ou refroidir le gaz provenant d'une source de distribution (61), et un circuit d'humidification (68) pour charger le gaz de plus ou moins d'humidité, en ce que les systèmes de contrôle et de régulation de la température et de l'humidité du produit en cours de culture comprennent au moins plusieurs capteurs de température (100, 101, 102) situés au niveau de la cuve (1), un capteur de température (115) et un capteur d'humidité relative (125) situés dans le circuit d'entrée (60a) du circuit (60) de circulation du gaz et des moyens de calcul (120) pour traiter les informations délivrées par lesdits capteurs (100,101, 102,115,125) et commander la batterie chaude (63), la batterie froide (64), le circuit d'humidification (68) et/ou des cycles de rotation de l'agitateur (17), et en ce qu'il comprend également des dispositifs de stérilisation de la cuve (1) et de l'ensemble des conduits du circuit (60) de circulation du gaz, de manière à ce que le procédé de fermentation puisse être conduit dans des conditions parfaitement stériles.

2. Réacteur selon la revendication 1, caractérisé en ce que la porte (10) de la cuve (1) est articulée autour d'une charnière (12) fixée à la cuve (1), ladite porte (10) supportant la grille (15), et en ce qu'un joint torique (13) assure l'étanchéité en position fermée de la porte (10).

3. Réacteur selon la revendication 1 ou 2, caractérisé en ce qu'un diffuseur de gaz (66) est branché sur l'ouverture d'entrée (65) de la porte (10) pour diffuser le gaz au travers de toute la surface de la grille (15).

4. Réacteur selon l'une quelconque des revendications précédentes, caractérisé en ce que l'agitateur (17) est animé d'un mouvement de rotation autour d'un premier axe vertical (X-X) passant par le centre de la cuve (1) et, simultanément, d'un mouvement de rotation sur lui-même autour d'un second axe vertical (Y-Y) passant par le centre de l'agitateur (17) situé sensiblement à mi-distance entre le premier axe vertical (X-X) et la paroi interne de la cuve (1).

5. Réacteur selon la revendication 4, caractérisé en ce que l'agitateur (17) est constitué de deux barres verticales (18 et 19), entretoisées, espacées l'une de l'autre d'une distance légèrement inférieure au rayon de la cuve (1) et réunies à leurs extrémités par deux tiges de liaison respectivement supérieure (20) et inférieure (21).

6. Réacteur selon la revendication 4 ou 5, caractérisé en ce que les mouvements de rotation de l'agitateur (17) sont assurés par un groupe moteur (M) dont l'arbre de sortie vertical (28) matérialise le premier axe de rotation (X-X), ledit premier arbre (28) étant accouplé en rotation à un système d'entraînement planétaire (29) qui supporte en rotation un second arbre vertical (35) qui matérialise le second axe de rotation (Y-Y) et qui est solidaire en rotation de l'agitateur (17), la transmission de mouvement entre les premier et second arbres (28 et 35) étant assurée par un pignon menant (28a), coaxial et solidaire du premier arbre (28) qui engrène un pignon mené (35a), coaxial et solidaire du second arbre (35).

7. Réacteur selon la revendication 6, caractérisé en ce que le groupe moteur (M) est supporté par le couvercle (11) de la cuve (1) et à l'extérieur de celle-ci, et en ce que le système d'entraînement planétaire (29) est logé dans la partie supérieure de la cuve (1).

8. Réacteur selon la revendication 1, caractérisé en ce que le système de contrôle de la température du produit en cours de culture comprend le capteur de température (102) qui est constitué par une sonde mobile destinée à mesurer la température au coeur du produit.

9. Réacteur selon la revendication 8, caractérisé en ce que le capteur de température (102) est solidaire de la tige de piston d'un vérin (110) monté à l'extérieur de la cuve (1).

10. Réacteur selon la revendication 9, caractérisé en ce que le capteur de température (102) traverse de part en part un élément tubulaire (105) rapporté sur la paroi latérale externe de la cuve (1) et dont une extrémité débouche à l'intérieur de la cuve (1) par une ouverture (104).

11. Réacteur selon l'une quelconque des revendications 8 à 10, caractérisé en ce que le système de contrôle de la température du produit en cours de culture comprend également les capteurs de température (100 et 101) qui sont respectivement situés en partie haute et en partie basse de la cuve (1) pour mesurer la température du produit à la périphérie de celui-ci.

12. Réacteur selon l'une quelconque des revendications 8 à 11, caractérisé en ce que le capteur de température (115) est supporté par la porte (10) de la cuve (1) pour mesurer la température du gaz injecté à l'entrée de la cuve (1).

13. Réacteur selon l'une quelconque des revendications 8 à 12, caractérisé en ce que le système de régulation de la température du produit en cours de culture s'effectue sur la valeur de la température du gaz à l'entrée de la cuve (1), ledit système comprenant la batterie chaude (63), la batterie froide (64), l'agitateur (17), un débitmètre massique (61) monté en sortie d'une source (60) de distribution du gaz, et en ce que la régulation de ladite température s'effectue en agissant sélectivement sur les éléments précités du système suivant les valeurs mesurées en continu par les capteurs de température (100, 101, 102, 115) et au moins une température de consigne.

14. Réacteur selon la revendication 1, caractérisé en ce que le système de contrôle du taux d'humidité relative du produit en cours de culture comprend le capteur d'humidité (125) qui est logé dans une chambre de mesure (CM1) montée dans le circuit d'entrée (60a) du circuit (60) de circulation de gaz, pour mesurer le taux d'humidité relative du gaz à l'entrée de la cuve (1).

15. Réacteur selon la revendication 14, caractérisé en ce que le système de régulation du taux d'humidité relative du produit en cours de culture s'effectue sur la valeur du taux d'humidité du gaz injecté à l'entrée de la cuve (1), ledit système comprenant le circuit d'humidification (68) pour charger le gaz de plus ou moins d'humidité, et en ce que la régulation dudit taux d'humidité est établie soit à partir de la valeur mesurée par ledit capteur (125) et d'une valeur de consigne, soit à partir de l'établissement d'un bilan massique.

16. Réacteur selon l'une quelconque des revendications précédentes, caractérisé en ce que la double enveloppe (3) de la cuve (1) communique avec l'extérieur par au moins deux ouvertures (4 et 5) sur lesquelles se raccordent les entrée-sortie d'un circuit (90) de circulation d'eau, par exemple, comprenant au moins un échangeur de chaleur (91), une pompe de circulation (P) et une chambre de mesure (CM3) dans laquelle est logé un capteur de température (92).

17. Réacteur selon l'une quelconque des revendications précédentes, caractérisé en ce que les dispositifs de stérilisation de la cuve (1) et de l'ensemble des conduits du circuit (60) de circulation du gaz, comprennent au moins un filtre d'entrée (F1) monté à la sortie de la source (61) de distribution du gaz située dans le circuit d'entrée (60a) du circuit (60) de circulation du gaz, un filtre de sortie (F2) monté dans le circuit de sortie (60b) du circuit (60) de circulation du gaz, et une entrée (V1) de vapeur d'eau située dans le circuit d'entrée (60a) du circuit (60) de circulation de gaz.

18. Réacteur selon la revendication 17, caractérisé en ce qu'un conduit de dérivation (c33), avec interposition d'une vanne de commande (V33), est branché en amont du filtre d'entrée (F1) et en aval de la chambre de mesure (CM1) où est logé un capteur (125) de mesure du taux d'humidité relative du gaz avant son entrée dans la cuve (1).

19. Réacteur selon l'une quelconque des revendications précédentes, caractérisé en ce que le couvercle (11) de la cuve (1) comprend également une ouverture d'entrée (55) de produits à l'intérieur de la cuve (1), ladite ouverture étant obturable par un bouchon de fermeture (56).

20. Réacteur selon la revendication 19, caractérisé en ce que le bouchon de fermeture (56) est traversé par un conduit d'entrée (57) par lequel peuvent être introduites des solutions, ledit conduit (57) étant associé à un dispositif de stérilisation comprenant un conduit (C51) relié à une entrée (V3) pour injecter de la vapeur d'eau dans le conduit d'entrée (57) et un conduit d'évacuation (C52) de ladite vapeur, une vanne (v50) étant montée dans le conduit d'entrée (57) pour isoler la cuve (1) dudit dispositif de stérilisation utilisé après chaque introduction d'une solution à l'intérieur de la cuve (1).

21. Réacteur selon l'une quelconque des revendications précédentes, caractérisé en ce qu'il comprend également une sonde (40) pour prélever, de façon stérile, une fraction du produit en cours de culture.

22. Réacteur selon la revendication 21, caractérisé en ce que la sonde (40) formée d'une tige (42) terminée, à une extrémité, par une coupelle de réception (43) et, à l'autre extrémité, par une collerette (44), ladite sonde (40) traversant une chambre (46) et un canal (47) respectivement formées dans deux pièces (48, 49), ladite pièce (49) étant fixée sur la cuve (1) et ladite pièce (48) étant fixée, de façon amovible, par un écrou (50) à ladite pièce (49).

23. Réacteur selon la revendication 22, caractérisé en ce que des moyens obturateurs (51a, 52a) sont respectivement logés dans ladite chambre (46) et dans ledit canal (47).

## Patentansprüche

1. Reaktor zur sterilen Durchführung von Verfahren zur Fermentation eines Produkts im festen Zustand, von der Bauart, die folgendes aufweist: einen zylindrischen Behälter mit einer aus einem doppelten Mantel gebildeten Seitenwand; eine Tür zum dichten Verschließen der unteren Stirnseite des Behälters; einen an dem Boden des Behälters angeordneten Rost; einen oberhalb des Rosts gelegenen rotierenden Rührer; einen Kreis für die Zirkulation eines Gases durch den Behälter; und Systeme zur Überwachung und Regelung der Temperatur und des Feuchtigkeitsgehalts des in Kultur befindlichen Produkts,
dadurch gekennzeichnet,
daß die obere Stirnseite des Behälters (1) durch einen Deckel (11) dicht geschlossen ist,
daß der Kreis (60) für die Zirkulation des Gases durch den Behälter (1) einen Eingangskreis (60a), der an eine in der Tür (10) vorgesehene Öffnung (65) angeschlossen ist, und einen Ausgangskreis (60b) aufweist, der an eine im Deckel (11) vorgesehene Öffnung (85) angeschlossen ist, wobei der Eingangskreis (60a) in Reihe mindestens eine heiße Batterie (63) und eine kalte Batterie (64) zum Erhitzen oder Kühlen des von einer Verteilerquelle (61) kommenden Gases und einen Befeuchtungskreis (68) zum Beladen des Gases mit mehr oder weniger Feuchtigkeit aufweist,
daß die Systeme zur Überwachung und Regelung der Temperatur und der Feuchtigkeit des in Kultur begriffenen Produkts mindestens mehrere in Höhe des Behälters (1) gelegene Temperaturfühler (100, 101, 102), einen Temperaturfühler (115) und einen Fühler (125) für die relative Feuchtigkeit, die sich im Eingangskreis (60a) des Gasumlaufkreises (60) befinden, und
eine Recheneinrichtung (120) zur Verarbeitung der von diesen Fühlern (100, 101, 102, 115, 125) gelieferten Daten und zur Steuerung der heißen Batterie (63), der kalten Batterie (64), des Befeuchtungskreises (68) und/oder der Drehzahl des Rührers (17) umfassen,
und daß er ferner Vorrichtungen zur Sterilisierung des Behälters (1) und der Gesamtheit von Leitungen des Gasumlaufkreises (60) aufweist, so daß das Fermentationsverfahren unter vollkommen sterilen Bedingungen durchgeführt werden kann.

2. Reaktor nach Anspruch 1,
dadurch gekennzeichnet,
daß die Tür (10) des Behälters (1) mit einem am Behälter (1) befestigten Scharnier (12) angelenkt ist, wobei diese Tür (10) den Rost (15) trägt,
und daß eine torusförmige Dichtung (13) die Abdichtung der Tür (10) in geschlossener Stellung gewährleistet.

3. Reaktor nach Anspruch 1 oder 2,
dadurch gekennzeichnet,
daß an die Eintrittsöffnung (65) der Tür (10) ein Gasverteiler (66) angeschlossen ist, der das Gas über die gesamte Oberfläche des Rosts (15) verteilt.

4. Reaktor nach einem der vorhergehenden Ansprüche,
dadurch gekennzeichnet,
daß der Rührer (17) mit einer Drehbewegung um eine erste vertikale Achse (X-X), die durch den Mittelpunkt des Behälters (1) verläuft, und gleichzeitig mit einer Drehbewegung um sich selbst um eine zweite vertikale Achse (Y-Y) angetrieben ist, die durch den Mittelpunkt des Rührers (17) verläuft, der sich im wesentlichen in der Mitte zwischen der ersten vertikalen Achse (X-X) und der Innenwand des Behälters (1) befindet.

5. Reaktor nach Anspruch 4,
dadurch gekennzeichnet,
daß der Rührer (17) aus zwei miteinander verstrebten vertikalen Stangen (18 und 19) besteht, deren Abstand voneinander etwas kleiner als der Radius des Behälters (1) ist und die an ihren Enden durch eine obere Verbindungsstange (20) bzw. eine untere Verbindungsstange (21) verbunden sind.

6. Reaktor nach Anspruch 4 oder 5,
dadurch gekennzeichnet,
daß die Drehbewegungen des Rührers (17) durch ein Antriebsaggregat (M) gewährleistet sind, dessen vertikale Ausgangswelle (28) die erste Drehachse (X-X) bildet, wobei diese erste Welle (28) rotationsmäßig mit einem Planetengetriebesystem (29) gekoppelt ist, das eine zweite drehbare vertikale Welle (35) trägt, die die zweite Drehachse (Y-Y) bildet und die rotationsmäßig mit dem Rührer (17) fest verbunden ist, wobei die Bewegungsübertragung zwischen der ersten und der zweiten Welle (28 und 35) mit einem zur ersten Welle (28) koaxialen und mit dieser fest verbundenen Antriebszahnrad (28a) gewährleistet ist, das mit einem zur zweiten Welle (35) koaxialen und mit dieser fest verbundenen Abtriebszahnrad (35a) kämmt.

7. Reaktor nach Anspruch 6,
dadurch gekennzeichnet,
daß das Antriebsaggregat (M) von dem Deckel (11) des Behälters (1) und außerhalb von diesen getragen ist und daß das Planetengetriebesystem (29) im oberen Teil des Behälters (1) untergebracht ist.

8. Reaktor nach Anspruch 1,
dadurch gekennzeichnet,
daß das System zur Überwachung der Temperatur des in Kultur begriffenen Produkts den Temperaturfühler (102) aufweist, der von einer beweglichen Sonde gebildet ist, die dazu bestimmt ist, die Temperatur im Inneren des Produkts zu messen.

9. Reaktor nach Anspruch 8,
dadurch gekennzeichnet,
daß der Temperaturfühler (102) mit der Kolbenstange einer außerhalb des Behälters (1) angebrachten Kolben-Zylinder-Einheit (110) fest verbunden ist.

10. Reaktor nach Anspruch 9,
dadurch gekennzeichnet,
daß der Temperaturfühler (102) ein rohrförmiges Element (105) vollständig durchquert, das an der äußeren Seitenwand des Behälters (1) angebracht ist und von dem das eine Ende durch eine Öffnung (104) in das Innere des Behälter (1) mündet.

11. Reaktor nach einem der Ansprüche 8 bis 10,
dadurch gekennzeichnet,
daß das System zur Überwachung der Temperatur des in Kultur begriffenen Produkts ferner die Temperaturfühler (100 und 101) umfaßt, die im oberen Teil bzw. im unteren Teil des Behälters (1) angeordnet sind, um die Temperatur des Produkts an dessen Umfang zu messen.

12. Reaktor nach einem der Ansprüche 8 bis 11,
dadurch gekennzeichnet,
daß der Temperaturfühler (115) von der Tür (10) des Behälters (1) getragen ist, um die Temperatur des am Eingang des Behälters (1) eingeleiteten Gases zu messen.

13. Reaktor nach einem der Ansprüche 8 bis 12,
dadurch gekennzeichnet,
daß das System zur Regelung der Temperatur des in Kultur begriffenen Produkts auf der Basis des Wertes der Temperatur des Gases am Eingang des Behälters (1) arbeitet, wobei das System die heiße Batterie (63), die kalte Batterie (64), den Rührer (17) und einen am Ausgang einer Quelle (61) zur Abgabe des Gases angeordneten Massendurchflußmesser (62) umfaßt, und daß die Regelung dieser Temperatur in der Weise vor sich geht, daß sie in Abhängigkeit von den von den Temperaturfühlern (100, 101, 102, 115) kontinuierlich gemessenen Werten und mindestens einer Solltemperatur selektiv auf die genannten Elemente des Systems einwirkt.

14. Reaktor nach Anspruch 1,
dadurch gekennzeichnet,
daß das System zur Überwachung des relativen Feuchtigkeitsgehalts des in Kultur begriffenen Produkts den Feuchtigkeitsfühler (125) aufweist, der in einer Meßkammer (CM1) angeordnet ist, die sich in dem Eingangskreis (60a) des Gasumlaufkreises (60) befindet, um den relativen Feuchtigkeitsgehalt des Gases am Eingang des Behälters (1) zu messen.

15. Reaktor nach Anspruch 14,
dadurch gekennzeichnet,
daß das System zur Regelung des relativen Feuchtigkeitsgehalts des in Kultur begriffenen Produkts auf der Basis des Wertes des Feuchtigkeitsgehalts des am Eingang des Behälters (1) eingeleiteten Gases arbeitet, wobei das System den Befeuchtungskreis (68) zum Beladen des Gases mit mehr oder weniger Feuchtigkeit umfaßt,
und daß die Regelung dieses Feuchtigkeitsgehalts entweder von dem von diesem Fühler (125) gemessenen Wert und einem Sollwert oder von der Erstellung einer Massenbilanz ausgeht.

16. Reaktor nach einem der vorhergehenden Ansprüche,
dadurch gekennzeichnet,
daß der doppelte Mantel (3) des Behälters (1) mit der Außenseite durch mindestens zwei Öffnungen (4 und 5) in Verbindung ist, an die der Eingang bzw. Ausgang beispielsweise eines Wasserumwälzkreises (90) angeschlossen ist, der mindestens einen Wärmetauscher (91), eine Umwälzpumpe (P) und eine Meßkammer (CM3) aufweist, in der ein Temperaturfühler (92) sitzt.

17. Reaktor nach einem der vorhergehenden Ansprüche,
dadurch gekennzeichnet,
daß die Vorrichtungen zur Sterilisierung des Behälters (1) und aller Leitungen des Gasumlaufkreises (60) mindestens ein Eingangsfilter (F1), das am Ausgang der im Eingangskreis (60a) des Gasumlaufkreises (60) vorgesehenen Gasabgabequelle (61) angeordnet ist, ein Ausgangsfilter (F2), das im Ausgangskreis (60b) des Gasumlaufkreises (60) angeordnet ist, und einen im Eingangskreis (60a) des Gasumlaufkreises (60) vorgesehenen Wasserdampfeinlaß (V1) umfaßt.

18. Reaktor nach Anspruch 17,
dadurch gekennzeichnet,
daß eine Zweigleitung (c33) mit eingesetztem Steuerventil (V33) stromaufwärts von dem Eingangsfilter (F1) und stromabwärts von der Meßkammer (CM1) angeschlossen ist, in der ein Fühler (125) zur Messung des relativen Feuchtigkeitsgehaltes des Gases vor seinem Eintritt in den Behälter (1) angeordnet ist.

19. Reaktor nach einem der vorhergehenden Ansprüche,
dadurch gekennzeichnet,
daß der Deckel (11) des Behälters (1) ferner eine Eingangsöffnung (55) für das Produkt in das Innere des Behälters (1) besitzt, die durch einen Verschlußstopfen (56) verschließbar ist.

20. Reaktor nach Anspruch 19,
dadurch gekennzeichnet,
daß der Verschlußstopfen (56) von einer Eintrittsleitung (57) durchsetzt ist, durch die Lösungen eingeführt werden können, wobei die Leitung (57) einer Sterilisierungsvorrichtung zugeordnet ist, die eine mit dem Eingang (V3) verbundene Leitung (C51) zum Einleiten des Wasserdampfes in die Eingangsleitung (57) und eine Leitung (C52) zur Abführung dieses Dampfes aufweist, wobei in der Eingangsleitung (57) ein Ventil (v50) angeordnet ist, um den Behälter (1) von dieser Sterilisierungsvorrichtung zu trennen, die nach jeder Einführung einer Lösung in das Innere des Behälters (1) verwendet wird.

21. Reaktor nach einem der vorhergehenden Ansprüche,
dadurch gekennzeichnet,
daß er außerdem eine Sonde (40) zur sterilen Entnahme eines Teils des in Kultur befindlichen Produkts besitzt.

22. Reaktor nach Anspruch 21,
dadurch gekennzeichnet,
daß die Sonde (40) aus einer Stange (42) besteht, die an dem einen Ende in einem Aufnahmetopf (43) und an dem anderen Ende in einem Flansch (44) endet, wobei diese Sonde (40) eine Kammer (46) und einen Kanal (47) durchquert, die in zwei Teilen (48, 49) vorgesehen sind, wobei das eine Teil (49) an dem Behälter (1) befestigt ist und das andere Teil (48) mit einer Mutter (50) an dem einen Teil (49) abnehmbar befestigt ist.

23. Reaktor nach Anspruch 22,
dadurch gekennzeichnet,
daß in der Kammer (46) und in dem Kanal (47) Absperreinrichtungen (51a, 52a) vorgesehen sind.

## Claims

1. Reactor for sterile conduction of processes of fermentation of a solid-state product, of the type which comprises a cylindrical vessel with a lateral wall which consists of a double envelope, a door which is intended to close in a sealed manner the lower end surface of the vessel, a grate which is disposed towards the base of the vessel, a rotary agitator which is disposed above the grate, a circuit for circulation of a gas through the vessel, and systems for controlling and regulating the temperature and level of humidity of the product being cultured, characterised in that the upper end surface of the vessel (1) is closed in a sealed manner by a cover (11), in that the circuit (60) for circulation of the gas through the vessel (1) comprises an intake circuit (60a) which is connected to an aperture (65) provided in the door (10), and an output circuit (60b) which is connected to an aperture (85) provided in the cover (11), the intake circuit (60a) comprising in series at least one preheater battery (63) and one cooling battery (64) to heat or cool the gas obtained from a distribution source (61), and a humidifying circuit (68) to charge the gas with a greater or lesser level of humidity, in that the systems for controlling and regulating the temperature and humidity of the product being cultured comprise at least several temperature sensors (100, 101, 102) which are disposed at the level of the vessel (1), a temperature sensor (115) and a relative humidity sensor (125) which are disposed in the intake circuit (60a) of the gas circulation circuit (60), and calculation means (120) to process the information provided by the sensors (100, 101, 102, 115, 125) and to control the preheater battery (63), the cooling battery (64), the humidifying circuit (68) and / or cycles of rotation of the agitator (17), and in that it also comprises devices for sterilisation of the vessel (1) and the assembly of the pipes of the circuit (60) for circulation of the gas, such that the fermentation process can be conducted in perfectly sterile conditions.

2. Reactor according to Claim 1, characterised in that the door (10) of the vessel (1) is articulated about a hinge (12) which is attached to the vessel (1), and the door (10) supports a grate (15), and in that an O-ring seal (13) seals the door (10) in the closed position.

3. Reactor according to Claim 1 or 2, characterised in that a gas distributor (66) is connected to the intake aperture (65) of the door (10), in order to distribute the gas across the entire surface of the grate (15).

4. Reactor according to any one of the preceding claims, characterised in that the agitator (17) is rotated about a first vertical axis (X-X) which passes through the centre of the vessel (1), and simultaneously is rotated about itself, around a second vertical axis (Y-Y) which passes through the centre of the agitator (17), and is disposed substantially half way between the first vertical axis (X-X) and the inner wall of the vessel (1).

5. Reactor according to Claim 4, characterised in that the agitator (17) consists of two braced vertical bars (18 and 19), which are spaced from one another by a distance which is slightly less than the radius of the vessel (1), and are connected at their ends by an upper connection rod (20) and a lower connection rod (21).

6. Reactor according to Claim 4 or 5, characterised in that the rotational movements of the agitator (17) are provided by a motor unit (M), the vertical output shaft (28) of which constitutes the first axis of rotation (X-X), the first shaft (28) being connected in a rotational manner to a planetary drive system (29) which supports in a rotational manner a second vertical shaft (35) which constitutes the second axis of rotation (Y-Y) and is rotationally integral with the agitator (17), transmission of motion between the first and second shafts (28 and 35) being provided by a driver pinion (28a) which is coaxial to, and integral with the first shaft (28), and engages a driven pinion (35a) which is coaxial to, and integral with the second shaft (35).

7. Reactor according to Claim 6, characterised in that the motor unit (M) is supported by the cover (11) of the vessel (1) and on the exterior of the latter, and in that the planetary drive system (29) is accommodated in the upper part of the vessel (1).

8. Reactor according to Claim 1, characterised in that the temperature control system for the product being cultured comprises the temperature sensor (102), which consists of a mobile probe intended to measure the temperature at the heart of the product.

9. Reactor according to Claim 8, characterised in that the temperature sensor (102) is integral with the piston rod of a jack (110) which is mounted on the exterior of the vessel (1).

10. Reactor according to Claim 9, characterised in that the temperature sensor (102) passes from one side to the other through a tubular component (105) which is provided on the outer lateral wall of the vessel (1), and of which one end leads inside the vessel (1) through an aperture (104).

11. Reactor according to any one of Claims 8 to 10, characterised in that the system for controlling the temperature of the product being cultured also comprises temperature sensors (100 and 101) which are disposed respectively on the upper and lower part of the vessel (1), in order to measure the temperature of the product on the periphery of the latter.

12. Reactor according to any one of Claims 8 to 11, characterised in that the temperature sensor (115) is supported by the door (10) of the vessel (1), in order to measure the temperature of the gas injected in the intake of the vessel (1).

13. Reactor according to any one of Claims 8 to 12, characterised in that the system for regulating the temperature of the product being cultured functions on the value of the temperature of the gas at the intake of the vessel (1), this system comprising the preheater battery (63), the cooling battery (64), the agitator (17), a mass flow meter (61) which is mounted at the output of a source (60) of distribution of the gas, and in that this temperature is regulated by acting selectively on the above-described components of the system, in accordance with the values measured continuously by the temperature sensors (100, 101, 102, 115) and at least one reference temperature.

14. Reactor according to Claim 1, characterised in that the system for controlling the level of relative humidity of the product being cultured comprises the humidity sensor (125) which is accommodated in a measuring chamber (CM1) which is mounted in the intake circuit (60a) of the gas circulation circuit (60), in order to measure the level of relative humidity of the gas at the intake of the vessel (1).

15. Reactor according to Claim 14, characterised in that the system for regulating the level of relative humidity of the product being cultured functions on the value of the level of humidity of the gas injected at the intake of the vessel (1), this system comprising the humidifying circuit (68) for charging the gas with a greater or lesser level of humidity, and this level of humidity is regulated either on the basis of the value measured by the sensor (125) and a reference value, or of a mass record produced.

16. Reactor according to any one of the preceding claims, characterised in that the double envelope (3) of the vessel (1) communicates with the exterior by means of at least two apertures (4 and 5) to which there are connected the intake-output of a circuit (90) for circulation of water for example, comprising at least a heat exchanger (91), a circulation pump (P) and a measuring chamber (CM3), in which a temperature sensor (92) is accommodated.

17. Reactor according to any one of the preceding claims, characterised in that the devices for sterilisation of the vessel (1) and the assembly of the pipes of the circuit (60) for circulation of the gas comprise at least an intake filter (Fl) which is mounted at the output of the source (61) of distribution of the gas which is provided in the intake circuit (60a) of the circuit (60) for circulation of the gas, an outlet filter (F2) which is mounted in the outlet circuit (60b) of the circuit (60) for circulation of the gas, and a water vapour intake (V1) which is provided in the intake circuit (60a) of the circuit (60) for circulation of the gas.

18. Reactor according to Claim 17, characterised in that a branch pipe (c33) with a control valve (V33) interposed is branched upstream of the intake filter (f1) and downstream of the measuring chamber (CM1) which contains a sensor (125) for measuring the level of relative humidity of the gas before it enters the vessel (1).

19. Reactor according to any one of the preceding claims, characterised in that the cover (11) of the vessel (1) also comprises an aperture (55) for the intake of products inside the vessel (1), the aperture being such that it can be sealed by a closing stopper (56).

20. Reactor according to Claim 19, characterised in that the closing stopper (56) is penetrated by an intake pipe (57) through which solutions can be introduced, the pipe (57) being associated with a sterilisation device which comprises a pipe (C51) connected to an intake (V3) for injection of the water vapour in the intake pipe (57) and a discharge pipe (C52) for this steam, a valve (v50) being mounted in the intake pipe (57) in order to isolate the vessel from this sterilisation device, which is used after each occasion that a solution is introduced into the vessel (1).

21. Reactor according to any one of the preceding claims, characterised in that it also comprises a sensor (40) for sterile collection of a fraction of the product being cultured.

22. Reactor according to Claim 21, characterised in that the sensor (40) consists of a rod (42) which ends at one end in a receiver dish (43) and at the other end in a collar (44), this sensor (40) passing through a chamber (46) and a channel (47) which are provided respectively in two parts (48, 49), the part (49) being attached to the vessel (1) and the part (48) being attached to the part (49) by means of a nut (50), such that it can be detached.

23. Reactor according to Claim 22, characterised in that the shutter means (51a, 52a) are accommodated respectively in the chamber (46) and the channel (47).
